# EUROPÄISCHE PATENTANMELDUNG

(11) **EP 1 438 984 A2**
(43) Veröffentlichungstag der Anmeldung: **21.07.2004**
(21) Anmeldenummer: 03028664.5
(22) Anmeldetag: 16.12.2003
(51) Int. Cl.: A61N 1/362, A61N 1/39

(54) **Bipolare Stimulationselektrode**

(30) Priorität: 20.01.2003 DE 10302319
(71) Anmelder: Osypka, Peter, Dr.-Ing., 79618 Rheinfelden-Herten (DE)
(72) Erfinder: Osypka, Peter, Dr.-Ing., 79618 Rheinfelden-Herten (DE)
(74) Vertreter: Maucher, Wolfgang, Dipl.-Ing.

(57) **Zusammenfassung**

Eine implantierbare bipolare Stimulationselektrode (1) hat eine im Herzen (2) zu fixierende Kathode (3) und eine dazu beabstandete Anode (4), die nahe oder an dem Stecker (5) angeordnet ist, mit welchem die Stimulationselektrode (1) mit einem Herzschrittmacher (7) verbunden ist, so dass sich die Anode (4) in Gebrauchsstellung außerhalb der Vene (8) befindet, durch welche die Zuleitung zu der Kathode (3) verläuft. Entsprechend flexibel kann die gesamte Elektrode und insbesondere auch der Bereich nahe der Kathode (3) ausgebildet sein.

## Beschreibung

Die Erfindung betrifft eine implantierbare bipolare Stimulationselektrode mit einer im Herzen zu fixierenden Katode, mit einer einen zweiten Pol bildenden und zu der Katode beabstandeten Anode und mit einem Stecker zum Verbinden der Stimulationselektrode mit einem implantierten Herzschrittmacher, wobei sich dieser Stecker am Herzschrittmacher außerhalb der zum Herzen führenden Vene befindet, durch die die Stimulationselektrode in Gebrauchsstellung verläuft.

Einer derartige Stimulationselektrode ist beispielsweise aus CH 653 559 A5 und aus US 5 545 205 A bekannt.

Bei der in der CH 653 559 A5 beschriebenen bipolaren Simulationselektrode sowie bei vergleichbaren derartigen Stimulationselektroden befindet sich der indifferente Pol, also die Anode, stets in einer Entfernung von etwa 10 mm bis 40 mm proximal vom Elektrodenkopf, der die Katode bildet. Da sowohl die Katode als auch die Anode aus festem, elektrisch leitfähigem Werkstoff bestehen, in der Regel Metall, und die indifferente Elektrode, also die Anode, stets eine erheblich größere Oberfläche als die Katode hat, also meist durch ein metallisches Rohr gebildet wird, ist nicht zu verhindern, dass der distale Teil einer derartigen bipolaren implantierbaren Stimulationselektrode auf eine Länge von etwa 20 mm bis 40 mm oder noch etwas mehr eine gewisse Steifigkeit aufweist.

Auch bei der in der US 545 205 beschriebenen Anordnung befindet sich die Stimulationselektrode in Gebrauchsstellung so nah am Elektrodenkopf, dass sie innerhalb des Herzens zu liegen kommt. Somit ergibt sich auch bei dieser Anordnung eine gewisse Steifigkeit der Stimulationselektrode.

Relativ steife Stimulationselektroden haben die Tendenz, nicht immer den durch die Herzkontraktion ausgelösten Bewegungen zu folgen, und verursachen dadurch eine gewisse Irritation der Herzinnenwand. Dadurch wird vermehrt Bindegewebe zwischen Elektrodenkopf und dem reizfähigen Gewebe der Herzinnenwand gebildet. Dies hat wiederum zur Folge, dass der Elektrodenkopf vom reizfähigen Gewebe abgehoben wird. Da sich die Reizschwelle mit dem Quadrat des Abstandes zwischen Elektrodenkopf und reizfähigem Gewebe verändert, muss mit einer Reizschwellenerhöhung gerechnet werden.

Die entsprechende Problematik wird verstärkt, wenn von einem Herzschrittmacher zwei Stimulationselektroden ausgehen, deren eine beispielsweise im Vorhof und deren andere im Ventrikel verankert wird, wofür bipolare Stimulationselektroden bevorzugt werden.

Durch die mit Abstand zu der Katode dieser benachbarte Anode ist außerdem die Stimulationselektrode bis zu der Anode mit einer entsprechenden Zuleitung zu versehen, so dass die Stimulationselektrode dadurch gegenüber einer unipolaren Elektrode eine verminderte Flexibilität hat.

In der US 3 915 174 ist eine Stimulationselektrode beschrieben, bei welcher sich die Anode in Gebrauchsstellung zwar außerhalb des Herzens aber innerhalb der zum Herzen führenden Vene befindet. Somit muss auch in diesem Falle über den bis zu dieser Anode führenden Bereich mit einer entsprechenden Steifigkeit der Stimulationselektrode gerechnet werden.

Es besteht deshalb die Aufgabe, eine bipolare Stimulationselektrode der eingangs genannten Art zu schaffen, die eine Zuleitung hoher Flexibilität bis zu der Katode hin aufweist.

Zur Lösung dieser Aufgabe ist die eingangs definierte Stimulationselektrode mit dem Stecker zum Verbinden mit dem Herzschrittmacher dadurch gekennzeichnet, dass die Anode nahe dem oder an dem Stecker auf dessen dem Herzschrittmacher abgewandter Seite derart angeordnet ist, dass sie sich in Gebrauchsstellung zwischen dem Stecker und dem Eintritt in die Vene befindet.

In überraschender Weise wird also die Anode mit erheblich größerer Entfernung zu der Katode angeordnet, so dass von dieser Anode aus die Zuleitung zu der Katode eine hohe Flexibilität haben kann und auch ihre Abmessungen vermindert sein können, weil eine Zuleitung zu der Anode in diesem Bereich nicht mehr erforderlich ist. Darüber hinaus entfällt im Inneren des Herzens die zu einer zusätzlichen Steifigkeit führende Anode, weil diese sich nun nahe dem Herzschrittmacher außerhalb der Vene befinden kann, wo ausreichend Platz dafür vorhanden ist, selbst wenn mehrere Stimulationselektroden von dem Herzschrittmacher ausgehen.

Beispielsweise kann die Anode unmittelbar benachbart zu einer Tülle des Steckers angeordnet sein. Solche auch als Knickschutz dienende Tüllen umschließen einen großen Teil des Steckers isolierend und geben nur den Steckerstift frei, der in einen entsprechenden Verbinder an dem Herzschrittmacher einsteckbar ist. Dieser den Stecker außenseitig umschließenden und isolierenden Tülle unmittelbar benachbart kann also die Anode der erfindungsgemäßen bipolaren Stimulationselektrode angeordnet sein, wodurch sie sich an einer Stelle befindet, die auch nach der Implantation noch außerhalb der Vene liegt, durch welche die Stimulationselektrode in das Herz verläuft.

Durch die erfindungsgemäße Anordnung der Anode nahe oder an dem Stecker des Herzschrittmachers ergibt sich ein Elektrodenschaft hoher Flexibilität, was zu einer geringeren mechanischen Irritation des Herzens und dadurch zu geringeren Reizschwellen führt. Ferner ist ein geringerer Schaftdurchmesser und damit auch ein Zugang durch dünnere Venen möglich. Dies ist vor allem dann von Vorteil, wenn eine Zweikammerstimulation gewünscht wird, bei welcher eine Stimulationselektrode im Vorhof und eine weitere Stimulationselektrode im Ventrikel verankert werden soll. Ferner ergeben sich keine Isolationsprobleme, wie sie bei den bisherigen relativ langen parallelen Zuleitungen zu Katode und Anode gelöst werden mussten. Außerdem ist der Materialverbrauch geringer, weil die Zuleitung zu der Anode kürzer ist, so dass auch die Herstellungskosten vermindert werden. Schließlich ergibt sich eine höhere Zuverlässigkeit, weil eine kürzere Zuleitung zu der Anode auch weniger bruchgefährdet ist.

Eine weitere Ausgestaltung der Erfindung von ganz erheblicher Bedeutung kann darin bestehen, dass auf der dem Herzschrittmacher und dem Stecker abgewandten Seite der Anode ein Aufnahmeteil, eine Kupplung oder ein Adapter oder dergleichen zum Verbinden mit dem proximalen Ende der Stimulationselektrode vorgesehen ist. Dadurch befindet sich die Anode ebenfalls in einem dem Stecker zu dem Herzschrittmacher benachbarten Bereich, jedoch kann die Zuleitung zum Herzen damit über einen weiteren Stecker verbunden sein, so dass gegebenenfalls auch eine Austausch möglich ist. Vor allem kann bei einem Schrittmacherwechsel jede bereits implantierte, bisher unipolare Elektrode in eine bipolare Elektrode umgewandelt werden, indem das dem Herzschrittmacher zugewandte Ende dieser unipolaren Elektrode mit dem Aufnahmeteil, der Kupplung oder dem Adapter verbunden wird, der zu der Anode führt. Dabei kann eine unipolare Stimulationselektrode mit einem nicht genormten Stecker ebenfalls in eine erfindungemäße bipolare Stimulationselektrode umgewandelt werden, indem man den bisher an dieser unipolaren Elektrode vorgesehenen Stecker abtrennt und die Wendel oder dergleichen nach dem Abisolieren mit einem Adapter mit Kupplung verbindet, wie es aus DE PS 39 06 598 bekannt ist.

Die Anode kann als außenseitig blanke Metallhülse insbesondere aus Platin, Platin-Iridium oder dergleichen gut leitendem Werkstoff bestehen. Da sie sich in Gebrauchsstellung an einer Stelle außerhalb der zu dem Herzen führenden Vene befindet, kann sie also entsprechend groß dimensioniert werden, ohne die Flexibilität der eigentlichen Stimulationselektrode zu beeinträchtigen.

Die Anode kann auch eine Loch-Anode sein, wobei sie als Wendel oder Hülse innerhalb einer gelochten Silikonummantelung angeordnet sein kann. Ferner kann die Anode als Wendel aus blankem Draht ausgebildet sein.

Um am Herzen mehrere Stimulationen durchführen zu können, können von einem Herzschrittmacher auch mehrere Elektroden ausgehen.

Es ist aber auch möglich, dass wenigstens zwei Anoden voneinander isoliert auf einem gemeinsamen Träger hintereinander angeordnet und wenigstens zwei im Herzen anbringbaren Katoden zugeordnet sind. Somit kann mit einem beispielsweise vierpoligen Stecker und einem einzelnen Träger für mehrere Anoden einer bipolaren Anordnung gesorgt werden, die platzsparend hintereinander, dem jeweiligen Stecker erfindungsgemäß benachbart angeordnet sein können.

Vor allem bei Kombination einzelner oder mehrerer der vorbeschriebenen Merkmale und Maßnahmen ergibt sich eine bipolare implantierbare Stimulationselektrode, deren durch eine Vene zum Herzen verlaufender Bereich und deren im Herzen verankertes Ende eine hohe Flexibilität wie eine unipolare Elektrode haben kann, so dass auch die Abmessungen vermindert sein können, ohne den Vorteil der bipolaren Ausbildung zu verlieren, so dass vor allem auch mehrere derartige bipolare Stimulationselektroden von einem gemeinsamen Herzschrittmacher ausgehen und mit unterschiedlichen Reizen arbeiten können.

Nachstehend sind Ausführungsbeispiele der Erfindung anhand der Zeichnung näher beschrieben. Es zeigt in zum Teil schematisierter Darstellung:
- Fig. 1: einen Herzschrittmacher mit einer erfindungsgemäßen Stimulationselektrode, die bis in den Ventrikel eines Herzens verläuft, wobei die Anode dieser bipolaren Stimulationselektrode dem Stecker des Herzschrittmachers unmittelbar benachbart ist,
- Fig. 2: eine der Fig.1 entsprechende Darstellung, bei welcher von dem Herzschrittmacher zwei erfindungsgemäße bipolare Stimulationselektroden ausgehen, deren eine im Ventrikel und deren andere im Vorhof des Herzens verankert sind, wobei jede dieser Stimulationselektroden einem Stecker zum Herzschrittmacher benachbart eine Anode aufweist, wobei die beiden Stecker und Anoden aus Platzgründen relativ zueinander derart versetzt sind, dass sie unterschiedliche Abstände von dem Herzschrittmacher haben,
- Fig. 3: in vergrößertem Maßstab die Ansicht einer bipolaren Stimulationselektrode gemäß dem Stand der Technik, bei welcher die Anode dem Stecker fern und der Katode benachbart angeordnet ist,
- Fig. 4: eine der Fig.3 entsprechende Darstellung einer erfindungsgemäßen Stimulationselektrode, bei welcher die als blanke Wendel ausgebildete Anode unmittelbar im Anschluss an eine Tülle des Steckers angeordnet ist,
- Fig. 5: eine Stimulationselektrode, bei welcher die wendelförmige Anode teilweise umschlossen ist,
- Fig. 6: eine Stimulationselektrode, bei welcher die Anode als blanke Hülse ausgebildet ist,
- Fig. 7: eine Stimulationselektrode, bei welcher die Anode als Loch-Anode ausgebildet ist, wobei der eigentlich leitende Teil als Wendel oder Hülse innerhalb eines gelochten Silikonteils angeordnet ist,
- Fig. 8: eine Ausführungsform der Erfindung, bei welcher die Anode an einem Adapter mit Aufnahmeteil vorgesehen ist, in den ein Stecker der eigentlichen Stimulationselektrode, beispielsweise einer unipolaren Elektrode, passt,
- Fig. 9: eine der Fig.8 vergleichbare Ausführungsform, bei welcher an dem die Anode aufweisenden Teil an dessen dem Stecker abgewandten Ende eine Kupplung zum Anschließen des abisolierten Endes der Elektrode, insbesondere einer unipolaren Elektrode vorgesehen ist,
- Fig. 10: eine Anordnung mit einem abgewandelten Adapter, bei welchem ein abisoliertes Ende der insbesondere unipolaren Stimulationselektrode durch den die Anode aufweisenden Teil und einen im Inneren befindlichen Kanal hindurch bis in einen hohlen Steckerstift einsteckbar und dort gemäß DE PS 39 06 598 verpressbar ist, sowie
- Fig. 11: eine erfindungsgemäße Stimulationselektrode mit zwei an einem gemeinsamen Träger hintereinander angeordneten, voneinander isolierten Anoden, die zwei hinter einer Verzweigung angebrachten Katoden zugeordnet sind, so dass zwei bipolaren Stimulationen an unterschiedlichen Stellen im Herzen durchführbar sind.

Eine im Ganzen mit 1 bezeichnete Stimulationselektrode weist eine im Herzen 2, beispielsweise im Ventrikel oder auch im Vorhof in beliebiger Weise zu fixierende Katode 3 auf, die also einen ersten Pol dieser bipolaren Stimulationselektrode bildet.

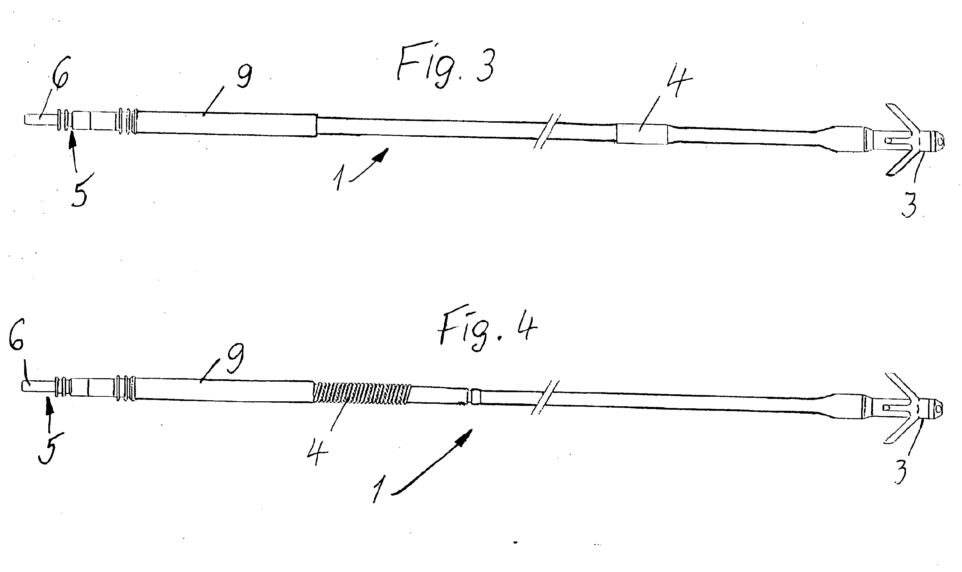

Die Stimulationselektrode 1 hat ferner eine einen zweiten Pol bildende und zu der Katode 3 beabstandete Anode 4, die gemäß Fig.3 im Stand der Technik einen relativ geringen Abstand zu der Katode 3 hatte und dadurch zu einer relativ steifen Stimulationselektrode 1 geführt hat.

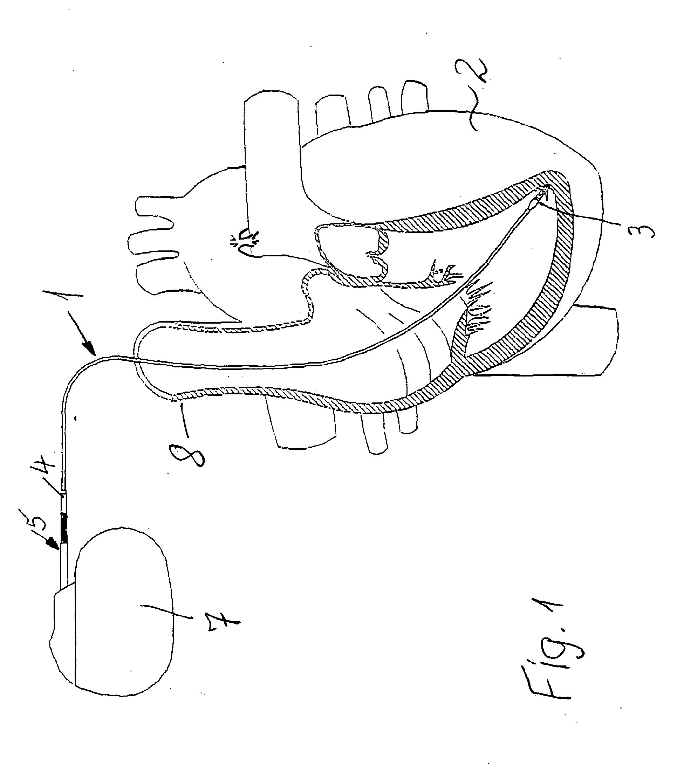

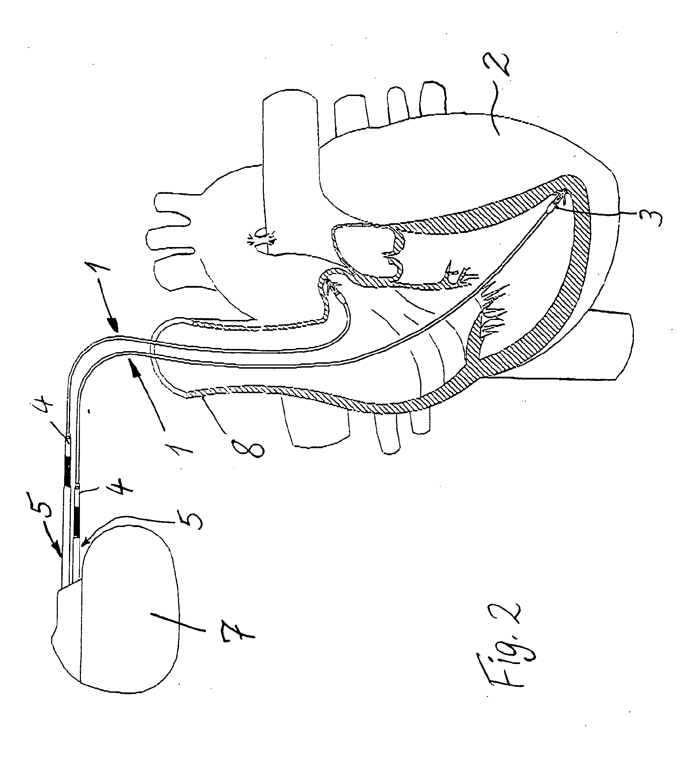

Zu der Stimulationselektrode 1 gehört ferner ein Stecker 5, dessen Steckerstift 6 in eine entsprechende Aufnahme oder Buchse an einem implantierbaren Herzschrittmacher 7 passt, so dass die Stimulationselektrode 1 mit Hilfe dieses Steckers 5 mit dem Herzschrittmacher 7 verbunden werden kann. Dabei befindet sich dieser Stecker 5 gemäß Fig.1 und 2 außerhalb der zum Herzen 2 führenden Vene 8, von der in Fig.1 und 2 nur ein kurzes, dem Herzen 2 nahes Stück dargestellt ist und durch welche die Stimulationselektrode 1 in Gebrauchsstellung verläuft.

Gemäß den Fig.1 und 2, sowie gemäß den Fig. 4 bis 10 ist die Anode 4 nahe dem oder an dem Stecker 5 auf dessen dem Herzschrittmacher 7 abgewandter Seite derart angeordnet, dass Sie sich in Gebrauchsstellung zwischen dem Stecker 5 und dem Eintritt in die Vene 8 befindet. Dadurch wird erreicht, dass die Anode 4 in Gebrauchsstellung außerhalb der Vene 8 angeordnet ist, so dass der von der Anode 4 zu der Katode 3 verlaufende Teil der Stimulationselektrode 1 dünner und flexibler gestaltet werden kann, weil in diesem Bereich keine Zuleitung zu der Anode 4 mehr benötigt wird. Somit kann die erfindungsgemäße bipolare Stimulationselektrode auch in relativ dünne Venen 8 eingeführt werden.

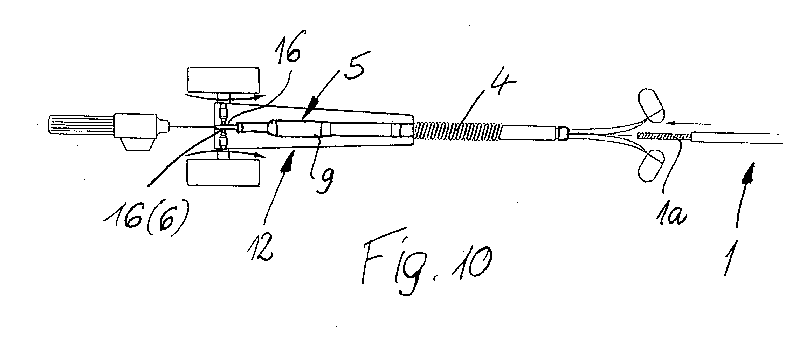

Während die Katode 3 bei Stimulationselektroden 1 gemäß dem Stand der Technik, wie in Fig.3 dargestellt, relativ nahe bei der Katode 3 angeordnet sind und somit ihre Zuleitung über nahezu die gesamte Länge der Stimulationselektrode 1 verlaufen muss, sind die verschiedenen Ausführungsformen der Anode 4 gemäß den Fig.4 bis 10 jeweils unmittelbar benachbart zu einer Tülle 9 des Steckers 5 angeordnet. Man erkennt dies besonders deutlich in den Fig.4, 8 und 9, in denen die Anode 4, wie im Ausführungsbeispiel nach Fig.10, als Wendel aus blankem Draht ausgebildet ist.

Besonders vorteilhaft wirkt sich diese Anordnung der Anode 4 bei solchen Fälle aus, bei denen mehrere Stimulationselektroden 1 von einem Herzschrittmacher 7 ausgehen und unterschiedliche Reize bewirken, so dass das Gehäuse des Herzschrittmachers nicht als Anode verwendbar wäre.

Dabei können die Stecker 5 und damit auch die Anoden 4 bei einer solchen Mehrfachanordnung von Stimulationselektroden 1 an dem selben Herzschrittmacher 7 dadurch gegeneinander versetzt sein, dass die Stecker 5 oder ihre Anschlüsse unterschiedliche Längen haben.

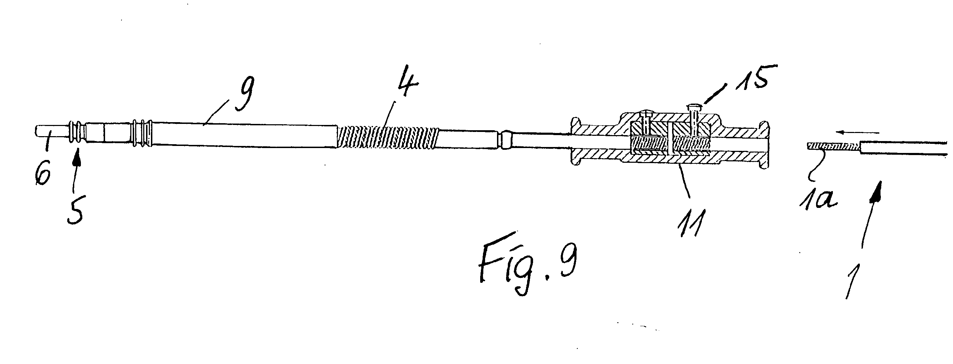

Besondere Ausführungsbeispiele zeigen die Fig.8 bis 10. In diesen Fällen ist vorgesehen, dass auf der dem Herzschrittmacher 7 und dem Stecker 5 abgewandten Seite der Anode 4 ein Aufnahmeteil 10 oder eine Kupplung 11 oder ein Adapter 12 oder dergleichen zum Verbinden mit dem proximalen, abisolierten Ende der Stimulationselektrode 1 vorgesehen ist, das heißt die Stimulationselektrode 1 hat zwischen der Anode 4 und der Katode 3 einen nachträglich verbindbare Unterbrechung.

Fig.8 zeigt dabei die Lösung mit einem Aufnahmeteil 10, in welches ein üblicher genormter Stecker 13 passt, wie er auch unmittelbar in einen Herzschrittmacher 7 eingesteckt werden könnte. Diese Anordnung erlaubt es, schon implantierte, bisher unipolar funktionierende Stimulationselektroden 1 bei einem Wechsel des Herzschrittmachers 7 durch das Einfügen der Anode 4 mit dem Aufnahmeteil 10 zu einer bipolaren Stimulationselektrode 1 zu machen.

Analoges gilt für die Ausführungsform nach Fig.9, bei welcher die Kupplung 11 einerseits mit einem Elektrodenteil verbunden ist, das zu der Anode 5 führt, während von der anderen Seite das abisolierte Ende 1a der Stimulationselektrode 1 einführbar und mit Klemmschrauben 15 fixierbar ist, und dann als Steckerstift 6 in den Herzschrittmacher 7 einführbar ist.

Eine dritte Möglichkeit, eine ursprünglich unipolare Stimulationselektrode in eine bipolare Stimulationselektrode 1 umzuwandeln, zeigt Fig.10, bei welcher das abisolierte Ende der Stimulationselektrode durch einen inneren freien Kanal der Anode 4 hindurchgeführt wird bis in einen in Inneren hohlen Steckerstift 16, der anschließend zusammengepresst wird, wie es aus US-Patent 5 050 602 und DE PS 39 06 598 bekannt ist.

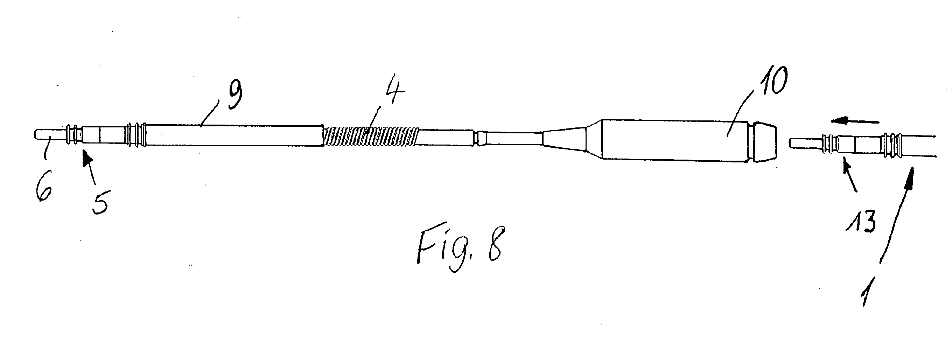

Während schon erwähnt wurde, dass die Anode 4 eine Wendel sein kann, zeigt Fig.6 die Möglichkeit, die Anode 4 als außenseitig blanke Metallhülse insbesondere aus Platin, Platin-Iridium oder dergleichen gut leitendem Werkstoff herzustellen.

Fig.7 zeigt eine Lösung, bei welcher die Anode 4 eine sogenannte Loch-Anode ist, wobei sie als Wendel oder Hülse innerhalb einer gelochten Silikonummantelung 17 angeordnet ist.

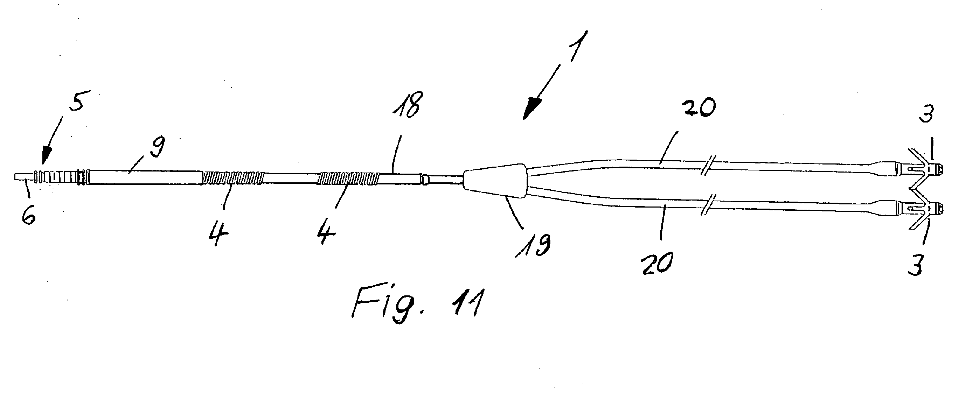

In Fig.11 ist eine Stimulationselektrode 1 dargestellt, die als Mehrfach-Elektrode bezeichnet werden könnte. Benachbart zu einem in diesem Falle vierpoligen Stecker 5 sind dabei an einem gemeinsamen Träger 18 zwei Anoden 4 voneinander isoliert hintereinander angeordnet und zwei im Herzen 2 anbringbaren Katoden 3 zugeordnet, wobei hinter dem Träger 18 eine Verzweigung 19 zu erkennen ist, die wie die Anoden 4 vor dem Eintritt in die entsprechenden Venen 8 in Gebrauchsstellung zu liegen kommt, so dass dann die eigentlichen Zuleitungen 20 zu den Katoden 3 ab der Verzweigung 19 einzeln "verlegt" sein können. Dadurch lässt sich eine ähnliche Anordnung und Stimulation bewirken, wie sie anhand von Fig. 2 erläutert ist, wobei aber ein einziger Stecker 5 mit entsprechend vielen Polen an dem Herzschrittmacher 7 einsteckbar ist.

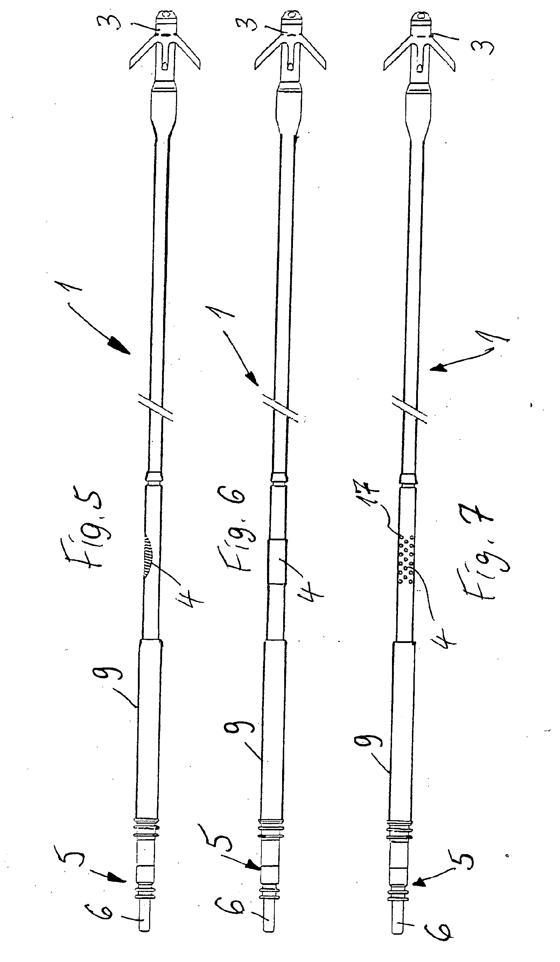

Die Anoden 4 sind im Ausführungsbeispiel nach Fig.11 als Wendeln ausgebildet, können aber auch anders gestaltet sein, beispielsweise analog Fig. 5, 6 oder 7.

Die implantierbare bipolare Stimulationselektrode 1 hat eine im Herzen 2 zu fixierende Katode 3 und eine dazu beabstandete Anode 4, die nahe oder an dem Stecker 5 angeordnet ist, mit welchem die Stimulationselektrode 1 mit einem Herzschrittmacher 7 verbunden ist, so dass sich die Anode 4 in Gebrauchsstellung außerhalb der Vene 8 befindet, durch welche die Zuleitung zu der Katode 3 verläuft. Entsprechend flexibel kann die gesamte Elektrode und insbesondere auch der Bereich nahe der Katode 3 ausgebildet sein.

## Patentansprüche

1. Implantierbare bipolare Stimulationselektrode (1) mit einer im Herzen (2) zu fixierenden Katode (3), mit einer einen zweiten Pol bildenden und zu der Katode (3) beabstandeten Anode (4) und mit einem Stecker (5) zum Verbinden der Stimulationselektrode (1) mit einem implantierten Herzschrittmacher (7), wobei sich dieser Stecker (5) am Herzschrittmacher außerhalb der zum Herzen (2) führenden Vene befindet, durch die die Stimulationselektrode (1) in Gebrauchsstellung verläuft, **dadurch gekennzeichnet, dass** die Anode (4) nahe dem oder an dem Stecker (5) auf dessen dem Herzschrittmacher (7) abgewandter Seite derart angeordnet ist, dass sie sich in Gebrauchsstellung zwischen dem Stecker (5) und dem Eintritt in die Vene (8) befindet.

2. Stimulationselektrode nach Anspruch 1, **dadurch gekennzeichnet, dass** die Anode (4) unmittelbar benachbart zu einer Tülle (9) des Steckers (5) angeordnet ist.

3. Stimulationselektrode nach Anspruch 1 oder 2, **dadurch gekennzeichnet, dass** auf der dem Herzschrittmacher (7) und dem Stecker (5) abgewandten Seite der Anode (4) ein Aufnahmeteil (10), eine Kupplung (11) oder eine Adapter (12) oder dergleichen zum Verbinden mit dem proximalen Ende der Stimulationselektrode (1) vorgesehen ist.

4. Stimulationselektrode nach einem der Ansprüche 1 bis 3, **dadurch gekennzeichnet, dass** die Anode (4) als außenseitig blanke Metallhülse insbesondere aus Platin, Platin-Iridium oder dergleichen gut leitendem Werkstoff besteht.

5. Stimulationselektrode nach einem der Ansprüche 1 bis 4, **dadurch gekennzeichnet, dass** die Anode (4) eine Loch-Anode ist, wobei sie als Wendel oder Hülse innerhalb einer gelochte Silikonummantelung (17)angeordnet ist.

6. Stimulationselektrode nach einem der Ansprüche 1 bis 5, **dadurch gekennzeichnet, dass** die Anode (4) als Wendel aus blankem Draht ausgebildet ist.

7. Stimulationselektrode nach einem der vorstehenden Ansprüche, **dadurch gekennzeichnet, dass** wenigstens zwei Anoden (4) voneinander isoliert dem Stecker (5) benachbart auf einem gemeinsamen Träger (18) hintereinander angeordnet und wenigstens zwei im Herzen (2) anbringbaren Katoden (3) zugeordnet sind.

8. Stimulationselektrode nach Anspruch 7, **dadurch gekennzeichnet, dass** am Ende des gemeinsamen Trägers (18) für die Anoden (4) eine Verzweigung (19) für die Zuleitungen (20) zu den Katoden (3) angeordnet ist.
